(19) Europäisches Patentamt
European Patent Office
Office européen des brevets

(11) Veröffentlichungsnummer: **0 119 516**
**B1**

# (12) EUROPÄISCHE PATENTSCHRIFT

(45) Veröffentlichungstag der Patentschrift: **20.09.89**

(51) Int. Cl.[4]: **C 07 D279/06**, A 61 K 7/48

(21) Anmeldenummer: **84102023.3**

(22) Anmeldetag: **27.02.84**

## (54) 3-Amino-tetrahydro-1,3-thiazin-2,4-dione, ihre Verwendung sowie diese Verbindungen enthaltende Hautbehandlungsmittel.

(30) Priorität: **16.03.83 DE 3309400**

(43) Veröffentlichungstag der Anmeldung: **26.09.84 Patentblatt 84/39**

(45) Bekanntmachung des Hinweises auf die Patenterteilung: **20.09.89 Patentblatt 89/38**

(84) Benannte Vertragsstaaten: **AT BE CH DE FR GB IT LI LU NL SE**

(56) Entgegenhaltungen:
**DE–A– 2 937 184**
**CHEMICAL ABSTRACTS, Band 87, 11,12. September 1977, p. 598, no. 84979h, Columbus, Ohio, USA**

(73) Patentinhaber: **Wella Aktiengesellschaft**
**Berliner Allee 65**
**D-6100 Darmstadt (DE)**

(72) Erfinder: **Hanefeld, Wolfgang, Prof. Dr.**
**Ernst-Lemmer-Strasse 63**
**D-3550 Marburg/Lahn (DE)**
Erfinder: **Röthlisberger, Rudi, Dr.**
**Chemin des Cossettes 2**
**CH-1723 Marly (CH)**
Erfinder: **Noser, Friedrich, Dr.**
**La Halta**
**CH-1711 Bonnefontaine (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft 3-Amino-tetrahydro-1,3-thiazin-2,4-dione sowie Hautbehandlungsmittel mit einem Gehalt an diesen Verbindungen.

Die Haut bildet die Grenzschicht zwischen dem Organismus und seiner Umwelt. Die wichtigste Aufgabe der Haut besteht deshalb darin, das Körperinnere gegen exogene Einflüsse zu schützen. Unsere Haut steht täglich mit körperfremden, zum Teil körper- und speziell hautfeindlichen, Stoffen in Kontakt. Besonders häufiger Kontakt der ungeschützten Haut mit diesen Stoffen, der oft berufsbedingt ist (Friseure, Zahnärzte, Hausfrauen), führt früher oder später zu mehr oder weniger schweren Hautschäden. Zur Verhinderung oder zumindest zur Verminderung dieser Hautschäden wurde bisher prinzipiell auf zwei Arten eingegriffen : Durch protektiven Hautschutz sowie durch konservierende Hautpflege.

Der protektive Hautschutz besteht darin, daß man die Haut vor dem Kontakt mit dem hautfremden Mittel behandelt, um damit den direkten Kontakt zwischen der Hautoberfläche und den schädigenden Stoffen weitgehend auszuschließen. Die Präparate, die einen protektiven Hautschutz gewähren, üben ihre Wirkung chemisch-physikalisch aus, ohne in die Physiologie der Haut einzugreifen. Solche Präparate müssen insbesondere die folgenden Anforderungen erfüllen : Sie sollen undurchlässig und unlöslich gegenüber den meisten exogenen Noxen sein ; sie sollen eine gute Hautverträglichkeit aufweisen ; sie sollen leicht aufzutragen und auch wieder von der Hautoberfläche zu entfernen sein ; sie sollen die Griffigkeit der Hände und damit die Arbeitsfähigkeit nicht beeinträchtigen und sollen eine gewisse Erhaltungsdauer aufweisen. Der Nachteil von bekannten Präparaten dieser Art liegt eindeutig darin, daß sie nicht in der Lage sind, alle diese Ansprüche optimal zu erfüllen.

Bei der konservierenden Hautpflege geht es darum, die Haut beim Kontakt mit hautschädlichen Mitteln weniger anfällig zu machen. Die Hautschutzstoffe sind bereits in den Waschmitteln enthalten. Man unterscheidet ihrer Wirkung nach verschiedene Arten von Hautschutzmaßnahmen, und zwar solche, die durch Adsorption an der Hautoberfläche wirken, rückfettende Maßnahmen, azidifizierende Maßnahmen und entquellende Maßnahmen. Auch hier besteht der wesentliche Nachteil dieser Hautschutzmaßnahmen darin, daß sie nicht gegen alle Aggressionen der verschiedenen Umweltnoxen in gleicher Weise wirksam sind.

In der deutschen Patentanmeldung DE-A-2 937 184 wurde über bestimmte 3-Amino-4-oxo-2-thioxo-tetrahydro-1,3-thiazinderivate berichtet. Sie werden dort zur Bekämpfung von Kopfschuppen empfohlen. Die 3-Amino-4-oxo-2-thioxo-tetrahydro-1,3-thiazine haben bei epicutaner Behandlung keine verdickende Wirkung auf die äußerste Hautschicht.

Demgegenüber erfüllen Hautbehandlungsmittel mit einem Gehalt an als Wirkstoff zur Verdickung der Oberhaut geeigneten 3-Amino-tetrahydro-1,3-thiazin-2,4-dionen der allgemeinen Formel I

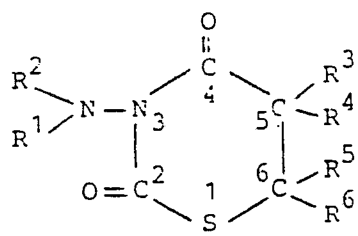

(I)

in der

$R^1$ und $R^2$ unabhängig voneinander H, Alkyl, Hydroxyalkyl, Carboxyalkyl, Halogenalkyl, Cyanoalkyl, Alkoxyalkyl, Cycloalkyl, Alkenyl, Alkinyl, Arylalkyl, Arylalkyl mit substituiertem Arylteil, Aryl, Alkyl-, Halogen-, Nitro-, Alkoxy-, Aryloxy-, Cyanosubstituiertes Aryl, Acyl, Thiazolyl, Thienyl, Benzthiazolyl, 1,3,4-Thiadiazolyl, Oxazolyl, Benzoxazolyl, 1,3,5-Oxadiazolyl, Pyrazolyl, 1,2,4-Triazolyl, Benzimidazolyl, Pyridinyl, Pyrimidinyl, Purinyl, Pyridazinyl, Triazinyl, Benzotriazinyl, Chinolyl, Isochinolyl, Cinnolinyl, Phthalazolyl, Pteridinyl, Chinoxazinyl, Acridinyl bedeuten, oder aber $R^1$ und $R^2$ gemeinsam für Alkyliden, Aryliden und Heterocyclyl-methyliden stehen, wobei der Heterocyclus Furan, Thiophen, Pyrrol, Pyridin, Isoxazol, Thiazol, Imidazol, 1,2,3-Triazol, Pyrazol, Indol, Benzthiazol, Chinolin, Isochinolin, Chinoxalin, Carbazol, Pteridin bedeutet, oder aber $R^1$ und $R^2$ zusammen das Segment $-(CH_2)_n-X-(CH_2)_m-$, mit $X = CH_2$, O, S, NR′ (R′ = Alkyl, Arylalkyl, Aryl), n = 0 bis 3 und m = 1 bis 3, darstellen, unter der Voraussetzung, daß n nur dann O ist, wenn $X = CH_2$ ist,

$R^3$ einen der Reste H, Alkyl, Cycloalkyl, Carboxyl, Arylalkyl, Aryl oder substituiertes Aryl darstellt,

$R^4$ die Bedeutung H, Alkyl, Cycloalkyl, Arylalkyl, Aryl oder substituiertes Aryl hat,

$R^5$ einer der Reste H, Alkyl, substituiertes Alkyl, Cycloalkyl, Arylalkyl, Aryl, substituiertes Aryl, 2-Furyl oder substituiertes 2-Furyl ist, und

$R^6$ einen der Reste H, Alkyl, substituiertes Alkyl, Cycloalkyl, Arylalkyl, Aryl oder substituiertes Aryl darstellt, infolge ihrer neuartigen Wirksamkeit alle Anforderungen, die an ein Hautschutzpräparat gestellt werden.

Im Rahmen dieser Anmeldung ist unter dem Begriff « Aryl » ein unsubstituierter aromatischer Kohlenwasserstoffrest zu verstehen.

Gegenstand der hier beschriebenen Erfindung sind daher Hautbehandlungsmittel, enthaltend physiologisch verträgliche Träger- und Zusatzstoffe, gekennzeichnet durch einem Gehalt an mindestens einer Verbindung der allgemeinen Formel I.

Die erfindungsgemäßen Hautbehandlungsmittel können in beliebigen, für Hautbehandlungsmittel geeigneten Zubereitungsformen, wie zum Beispiel als klare, gefärbte oder trübe Lösung, als Dispersion, Emulsion, in Form eines Schaumes oder aber als eine aus einem Aerosolbehälter mittels einer Pumpe oder durch ein Treibgas zu versprühende Zubereitung vorliegen. Bevorzugt liegen sie jedoch als Salbe, Creme oder Gel vor. Als Beispiele für erfindungsgemäß in Betracht kommende Zubereitungen seien insbesondere kosmetische Hautbehandlungsmittel, wie Tagescremes, Nachtcremes, Nährcremes, Hautschutzcremes, Sonnenschutzcremes, Sonnenschutzsprays, Kälteschutzcremes sowie weiterhin Lippenstifte, Hautmilch-Zubereitungen, Hautlotionen und Hautschutzgele genannt.

Die Konzentration der Verbindungen der allgemeinen Formel I beträgt in den Hautbehandlungsmitteln insgesamt etwa 0,1 bis 5 Gew. %, vorzugsweise 0,5 bis 3 Gew. %. Hierbei können die Verbindungen der Formel I jeweils alleine oder im Gemisch miteinander in den Mitteln vorliegen.

Die Zusammensetzung der Hautbehandlungsmittel stellt eine Mischung der Verbindungen gemäß Formel I mit den für solche Zubereitungen üblichen physiologisch verträglichen Bestandteilen, wie Träger- und Zusatzstoffen, dar.

Übliche Träger- und Zusatzstoffe in Lösungen, Cremes, Emulsionen oder Gelen sind zum Beispiel Lösungsmittel wie Wasser, niedere aliphatische Alkohole, beispielsweise Ethanol, Propanol und Isopropanol, oder Glykole wie Glycerin und Propylenglykol, weiterhin Netzmittel oder Emulgatoren aus den Klassen der anionischen, kationischen, amphoteren oder nichtionogenen oberflächenaktiven Substanzen wie Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Fettsäureester, Stärke, Cellulosederivate, Vaseline, Stearin, Ceresin, Paraffinöl und Fettsäuren sowie außerdem Pflegestoffe wie Lanolin, Lanolinderivate, Cholesterin, Pantothensäure, Sorbit, Betain, Mandelöl, Avocadoöl, Bienenwachs und Walrat.

Weitere übliche Zusatzstoffe sind zum Beispiel kosmetische Harze, Farbstoffe, Parfümöle, Treibgase sowie Konservierungsstoffe, wie z. B. p-Hydroxybenzoesäure, Sorbinsäure, Salicylsäure, Formaldehyd und Hexachlorophen. Zur Salzbildung können die Mittel Basen, wie z. B. Triethanolamin, enthalten.

Die Herstellung der Hautbehandlungsmittel erfolgt in der für derartige Präparate üblichen Weise, indem die als Wirkstoff dienenden Verbindungen der Formel I mit den für die Hautbehandlungsmittel als Trägerstoff dienenden Bestandteilen vermischt werden und danach mit den weiteren Bestandteilen der Mittel zum fertigen Endprodukt konfektioniert werden. Die als Bestandteil der hier beschriebenen Hautbehandlungsmittel enthaltenen Verbindungen der allgemeinen Formel I bewirken nach wiederholter epicutaner Behandlung eine Verdickung der äußersten Hautschicht, nämlich der toten Hornschicht, welche ja in erster Linie für den natürlichen Hautschutz verantwortlich ist. Durch diese Verdickung der Hornschicht wird die Haut gegen den Kontakt mit Umweltnoxen jeder Art widerstandsfähiger und bildet auf diese Art und Weise den optimalen Hautschutz. Da der Hautschutz, der durch diese Verbindungen gewährleistet wird, darin besteht, daß der natürliche Hautschutz verstärkt wird, weisen die erfindungsgemäßen Hautschutzmittel, welche Verbindungen nach der Formel I enthalten, die Nachteile, welche bei konventionellen Hautschutzmitteln beobachtet werden, nicht auf. Die erfindungsgemäßen Hautbehandlungsmittel können zeitlich unabhängig vom Kontakt mit der hautfremden Noxe auf die Hautoberfläche aufgetragen werden. Sie werden deshalb niemals einen Arbeitsvorgang stören, da sie sich zu diesem Zeitpunkt nicht mehr auf der Hautoberfläche befinden. Der Hautschutz kann auch nicht entfernt werden (z. B. durch Waschen), da er ja durch den neuen Zustand der Haut (Verdickung) erreicht wird. Gleichzeitig bewirken die Verbindungen der allgemeinen Formel I eine Verstärkung des natürlichen Sonnenschutzes. Dieser zusätzliche Sonnenschutz wird ebenfalls durch die Verdickung der Hornschicht, die nach der Behandlung mit dem erfindungsgemäßen Hautbehandlungsmittel erzielt wird, erreicht. Eine Verdickung der Hornschicht bewirkt nämlich eine erhöhte Absorption der Licht- bzw. Sonnenstrahlen. Dieser neuartige prophylaktische Sonnenschutz (Prä-sun) weist gegenüber der mit den konventionellen Sonnenschutzmitteln erzielbaren Wirkung eindeutig Vorteile auf. Die üblichen Sonnenschutzmittel werden auf die Hautoberfläche aufgetragen, und dabei ist ihre Absorptionskapazität, d. h. ihre Lichtschutzfähigkeit, von der aufgetragenen Schichtdicke abhängig. Diese Präparate können stören, indem sie z. B. zu stark fetten und dadurch Kleider verschmutzen. Sie werden z. B. durch Baden oder Duschen wieder abgespült und müssen deshalb ständig neu aufgetragen werden. Die erfindungsgemäßen Mittel können dagegen zeitlich unabhängig von der Exposition an der Sonne aufgetragen werden, indem sie, z. B. schon 3 bis 4 Wochen vor einem Sommer-Urlaub, wiederholt epicutan appliziert werden, so daß sie zur Urlaubszeit dann, dank der verdickten Hornschicht, schon einen langdauernden, nicht abwaschbaren Schutz vor Sonnenbrand und sonstigen chronischen Lichtschäden bieten.

In ähnlicher Weise sind die erfindungsgemäßen Mittel durch ihren Gehalt an den 3-Amino-tetrahydro-1,3-thiazin-2,4-dionen der Formel I in der Lage, an exponierten und empfindlichen Körperstellen, wie z. B. an Gesicht und Händen, die schlechten Witterungsverhältnissen besonders ausgesetzt sind, einen Schutz vor Kälte zu bieten. Die Mittel kommen daher insbesondere auch für die Anwendung durch Skiläufer und

Hochgebirgssportler, beispielsweise als vorbeugender Schutz gegen extreme Kälte, in Betracht. Dank ihrer hornschichtverdickenden Wirkung sind die erfindungsgemäßen Hautbehandlungsmittel in der Lage, einen wirksamen Kälteschutz zu bieten, der auch in diesem Falle nicht die bei hautbelastenden und auf der Hautoberfläche verbleibenden Präparaten unangenehm empfundenen Nebenwirkungen aufweist, denn auch hier handelt es sich lediglich um eine Verstärkung des natürlichen Kälteschutzes.

Mit zunehmendem Alter wird die äußere Schicht der Haut, die sogenannte Oberhaut oder Epidermis, immer dünner. Die Verdünnung der Epidermis ist dafür verantwortlich, daß die Hautoberfläche mit dem Alter ihr typisches pergamentartiges Aussehen erhält und daß Talgdrüsen, Retentionszysten, Pigmentflecken sowie feine Blutgefäße sichtbarer werden und die typische Beschaffenheit einer sogenannten Altershaut prägen. Indem die erfindungsgemäßen Mittel in der Lage sind, nicht nur die Hornschicht, die ja nur einen Teil der Epidermis darstellt, sondern die gesamte Epidermis zu verdicken, stellen sie ein wirksames Mittel zur prophylaktischen Behandlung der Hautalterung dar.

Die erfindungsgemäßen Hautbehandlungsmittel werden zweckmäßig in der Weise angewendet, daß sie beginnend etwa 3 bis 4 Wochen vor dem Zeitpunkt, zu dem eine Verdickung der Hornhaut bzw. der Epidermis vorliegen soll, wiederholt und vorzugsweise 1 bis 2-mal täglich auf die entsprechenden Hautbereiche aufgetragen werden.

Die hautverdickende Wirkung der erfindungsgemäßen Verbindungen wurde an haarlosen Mäusen folgendermaßen nachgewiesen :

Jeweils 2 Gew. % der Verbindungen a, e und h wurden in Form einer 50-volumenprozentigen ethanolischen Lösung während zweieinhalb Wochen, täglich, außer Samstag und Sonntag, auf die eine Körperseite von haarlosen Mäusen (hr/hr) epicutan appliziert. Am Ende der Behandlungszeit wurden die Tiere getötet, auf beiden Körperseiten eine ca. 1 × 1,5 cm große Hautfläche entnommen und histologisch bearbeitet. Die Dicke der Oberhaut wurde an ca. 100 Stellen ausgemessen und die durchschnittliche Hautdicke ermittelt. Die Verdickung der Oberhaut ergab sich dann durch den Quotienten aus der durchschnittlichen Dicke der behandelten Oberhaut und der durchschnittlichen Dicke der unbehandelten Haut. Dieser Quotient wird als Verdickungsfaktor bezeichnet. Die erfindungsgemäßen Verbindungen ergaben Verdickungsfaktoren, die zwischen 1,2 und 2,1 lagen.

Die in den hier beschriebenen Hautbehandlungsmitteln enthaltenen erfindungsgemäßen 3-Amino-tetrahydro-1,3-thiazin-2,4-dione der Formel I sind neu.

Beispiele für erfindungsgemäße neue Verbindungen der Formel I sind

a) 3-Dimethylamino-5,5-diphenyl-tetrahydro-1,3-thiazin-2,4-dion
b) 3-(N-Acetyl-N-phenyl)-amino-5,5-diphenyl-tetrahydro-1,3-thiazin-2,4-dion
c) 3-[N-Acetyl-N-(4-chlorphenyl)]-amino-5,5-diphenyl-tetrahydro-1,3-thiazin-2,4-dion
d) 3-[N-Acetyl-N-(4-nitrophenyl)]-amino-5,5-diphenyl-tetrahydro-1,3-thiazin-2,4-dion
e) 3-Diphenylamino-5,5-diphenyl-tetrahydro-1,3-thiazin-2,4-dion
f) 3-Dimethylamino-5,5-di-(4-tolyl)-tetrahydro-1,3-thiazin-2,4-dion
g) 3-(1-Perhydroazepinyl)-tetrahydro-1,3-thiazin-2,4-dion
h) 3-(4-Morpholinyl)-tetrahydro-1,3-thiazin-2,4-dion
i) 3-(1-Perhydroazepinyl)-5,5-di-(4-tolyl)-tetrahydro-1,3-thiazin-2,4-dion

Die Herstellung der Verbindungen der Formel I erfolgt durch Oxidation des entsprechenden 3-Amino-tetrahydro-2-thioxo-1,3-thiazin-4-ons mit Chromsäureanhydrid in Essigsäure. Die Herstellung dieser Ausgangsverbindungen ist in der deutschen Offenlegungsschrift 2 937 184 sowie in der Literatur W. Hanefeld et al., Archiv der Pharmazie, Weinheim, 315 (2) Seiten 103-119 (1982) beschrieben.

Das dort beschriebene Verfahren zur Herstellung der 3-Amino-4-oxo-2-thioxo-tetrahydro-1,3-thiazine der Formel II besteht darin, daß man Hydrazin, ein N-mono-substituiertes Hydrazin, ein N,N-disubstituiertes Hydrazin, die Salze dieser Verbindungen oder Hydrazone von Aldehyden oder Ketonen in einem geeigneten polaren Lösungsmittel, vorzugsweise in Ethanol/Pyridin, mit Schwefelkohlenstoff und einer Base, vorzugsweise Natronlauge, Kalilauge oder einem tertiären Amin, zum Dithiocarbazat reagieren läßt, anschließend mit einem β-Lacton zum Salz des Dithiocarbazidsäure-2-carboxyethylesters umsetzt und nach dem Entfernen des Lösungsmittels den Ester entweder A) durch Erhitzen mit Acetanhydrid und einigen Tropfen konzentrierter Schwefelsäure direkt zum 3-Amino-4-oxo-2-thioxo-tetrahydro-1,3-thiazin cyclisiert oder B) durch Zugabe von Salzsäure zunächst in die freie Säure überführt und erst dann mit Acetanhydrid/Schwefelsäure zum 3-Amino-4-oxo-2-thioxo-tetrahydro-1,3-thiazin cyclisiert.

Das Verfahren sei durch das folgende Reaktionsschema, in dem $R^1$, $R^2$, $R^3$, $R^4$, $R^5$ und $R^6$ die vorstehend angegebene Bedeutung haben, näher erläutert :

(Siehe Tabelle Seite 5 f.)

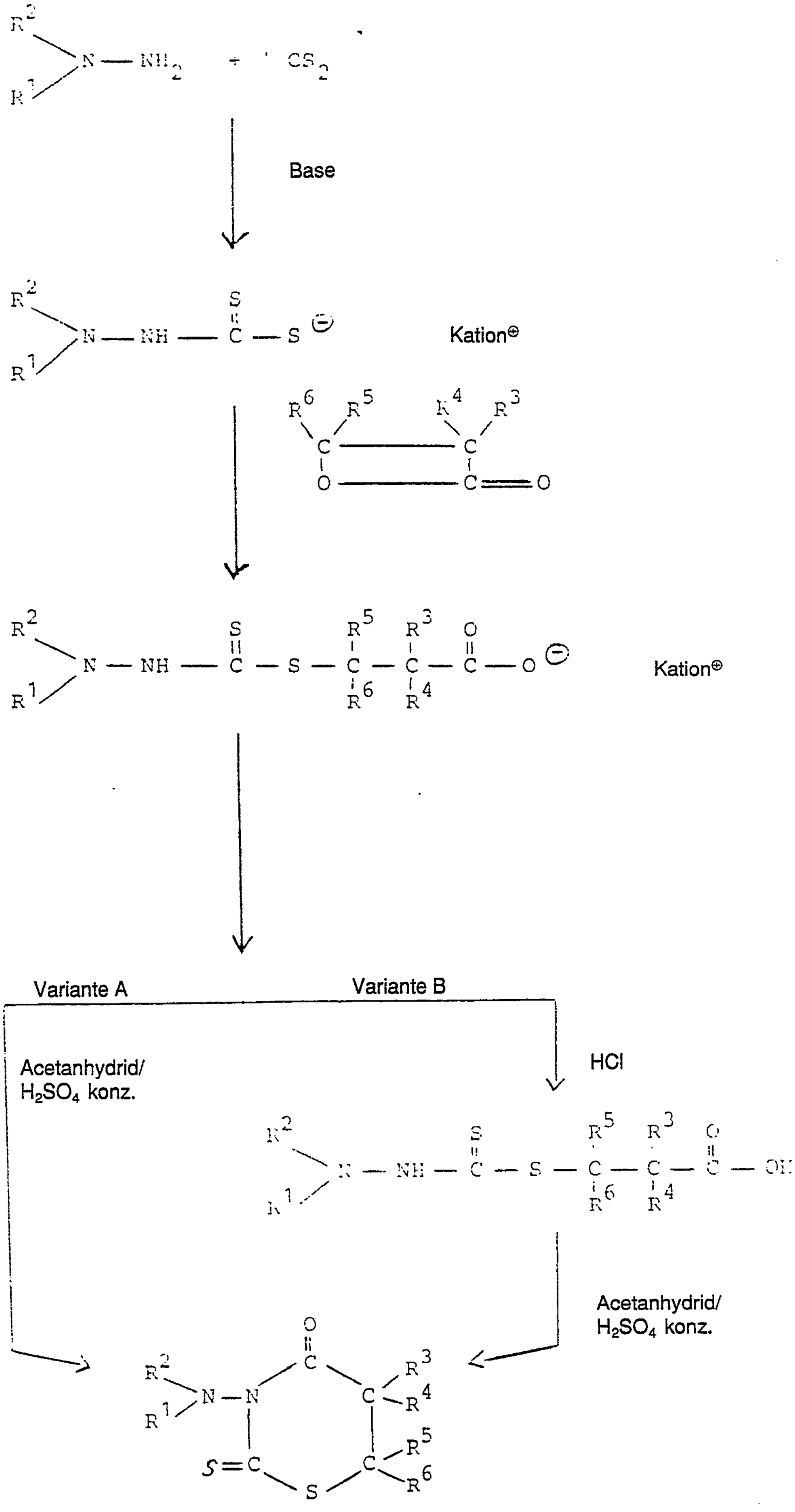

Base
Kation⊕
Kation⊕
Variante A
Variante B
Acetanhydrid/
$H_2SO_4$ konz.
HCl
Acetanhydrid/
$H_2SO_4$ konz.


(II)

Zur Herstellung der erfindungsgemäßen 3-Amino-tetrahydro-1,3-thiazin-2,4-dione der Formel I wird das entsprechende 3-Amino-tetrahydro-2-thioxo-1,3-thiazin-4-on der Formel II in der etwa 10-fachen Gewichtsmenge Essigsäure gelöst und gemäß nachstehender Reaktionsgleichung mit der 3-fachen Molmenge Chromsäureanhydrid unter 1- bis 2-stündigem Erhitzen am Rückfluß zum gewünschten Produkt der Formel I umgesetzt.

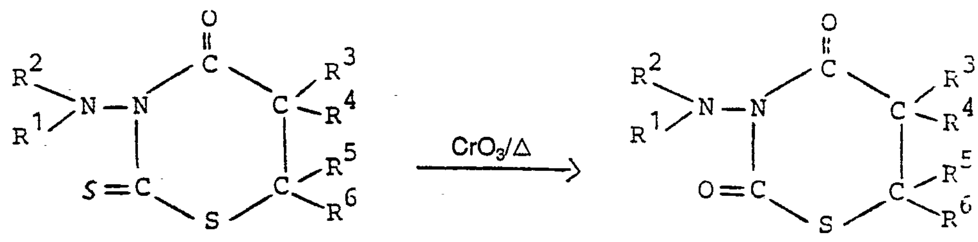


(II) (I)

Das Reaktionsgemisch wird anschließend bis zur beginnenden Trübung mit Wasser versetzt und sodann abgekühlt. Die abgeschiedenen Kristalle des 3-Amino-tetrahydro-1,3-thiazin-2,4-dions werden abgesaugt, mit Wasser gewaschen, getrocknet und anschließend mit einem geeigneten Lösungsmittel, wie zum Beispiel Toluol, einem Gemisch aus Aceton/Wasser oder Diethylether/Petrolether, umkristallisiert.

Die nachstehenden Beispiele sollen den Gegenstand der Erfindung näher erläutern.

## Beispiele für Hautbehandlungsmittel

Präparate für den Hautschutz

Lotion

| | |
|---|---|
| 2,5 g | 3-Dimethylamino-5,5-diphenyl-tetrahydro-1,3-thiazin-2,4-dion |
| 3,5 g | Glycerinmonostearat, selbstemulgierend |
| 2,0 g | Ölsäure |
| 5,0 g | Glycerin |
| 1,0 g | Triethanolamin |
| 5,0 g | Ethanol (96 Vol. %-ig) |
| 0,3 g | Parfüm und Konservierungsmittel |
| 80,7 g | Wasser |
| 100,0 g | |

Gel

| | |
|---|---|
| 3,0 g | 3-[N-Acetyl-N-(4-Chlorphenyl)]-amino-5,5-diphenyl-tetrahydro-1,3-thiazin-2,4-dion |
| 15,0 g | Ethanol (96 Vol. %-ig) |
| 1,0 g | Acrylsäurehomopolymerisat |
| 10,0 g | Glycerin |
| 0,8 g | Triethanolamin |
| 0,4 g | Parfüm und Konservierungsmittel |
| 69,8 g | Wasser |
| 100,0 g | |

Prä-sun-Präparate (Sonnenschutzmittel)

Lotion

| | |
|---|---|
| 2,0 g | 3-Diphenylamino-5,5-diphenyl-tetrahydro-1,3-thiazin-2,4-dion |
| 3,0 g | Glycerinmonostearat, selbstemulgierend |
| 3,0 g | Isopropylmyristat |
| 2,0 g | Glycerin |
| 0,3 g | Parfüm und Konservierungsmittel |
| 89,7 g | Wasser |
| 100,0 g | |

Emulsion

| | |
|---|---|
| 2,5 g | 3-Dimethylamino-5,5-diphenyl-tetrahydro-1,3-thiazin-2,4-dion |
| 3,0 g | Glycerinmonostearat |
| 2,0 g | Cetyl-Stearylalkohol (DAB 7) |
| 1,5 g | Cetyl-Stearylalkohol, mit 12 Mol Ethylenoxid oxethyliert |
| 1,5 g | Cetyl-Stearylalkohol, mit 20 Mol Ethylenoxid oxethyliert |
| 0,5 g | Hydriertes Rizinusöl |
| 10,0 g | 2-Octyldodecanol |
| 6,0 g | Paraffinöl, dickflüssig |
| 0,4 g | Parfüm und Konservierungsmittel |
| 72,6 g | Wasser |
| 100,0 g | |

Präparate für den prophylaktischen Kälteschutz

Milch

| | |
|---|---|
| 2,0 g | 3-[N-Acetyl-N-(4-nitrophenyl)]-amino-5,5-diphenyl-tetrahydro-1,3-thiazin-2,4-dion |
| 5,8 g | Sorbitanmonostearat |
| 2,2 g | Sorbitanmonooleat |
| 8,0 g | Mandelöl |
| 5,0 g | Ölsäuredecylester |
| 3,0 g | 2-Octyldodecanol |
| 6,0 g | Perhydrosqualen |
| 2,0 g | Glycerin |
| 1,8 g | Propylenglykol |
| 3,0 g | Parfüm und Konservierungsmittel |
| 61,2 g | Wasser |
| 100,0 g | |

Gel

| | |
|---|---|
| 3,0 g | 3-Diphenylamino-5,5-diphenyl-tetrahydro-1,3-thiazin-2,4-dion |
| 15,0 g | Ethanol (96 Vol. %-ig) |
| 1,0 g | Acrylsäurehomopolymerisat |
| 10,0 g | Glycerin |
| 0,8 g | Triethanolamin |
| 0,4 g | Parfüm und Konservierungsmittel |
| 69,8 g | Wasser |
| 100,0 g | |

Präparate zur prophylaktischen Behandlung der Altershaut

Nährcreme

| | |
|---|---|
| 1,5 g | 3-Dimethylamino-5,5-diphenyl-tetrahydro-1,3-thiazin-2,4-dion |
| 25,0 g | 1 :1 Kondensat aus Zitronensäure-di-stearylester und Pentaerytrit-di-kokosfettsäureester |
| 10,0 g | Ölsäuredecylester |
| 10,0 g | Isopropylmyristat |
| 0,3 g | Parfüm und Konservierungsmittel |
| 53,2 g | Wasser |
| 100,0 g | |

Antifalten-Creme

| | |
|---|---|
| 2,5 g | 3-[N-Acetyl-N-(4-chlorphenyl)]-amino-5,5-diphenyl-tetrahydro-1,3-thiazin-2,4-dion |
| 7,0 g | Glycerinmonopalmitatdistearat |
| 7,0 g | Stearinsäure, dreifach gepreßt |
| 1,5 g | Jojobaöl |
| 5,0 g | Isopropylmyristat |

2,0 g Glycerin
0,9 g Triethanolamin
3,0 g Parfüm und Konservierungsmittel
71,1 g Wasser

100,0 g

Herstellungsbeispiele

Herstellung vom 3-Dimethylamino-5,5-diphenyl-tetrahydro-1,3-thiazin-2,4-dion (a) :

6,85 g (20 mMol) 3-Dimethylamino-5,5-diphenyl-2-thioxo-tetrahydro-1,3-thiazin-4-on werden in 70,0 g Essigsäure unter Erwärmen gelöst und mit 6,0 g (60 mMol) $CrO_3$ versetzt. Nach Abklingen der heftig exothermen Reaktion wird eine Stunde zum Sieden erhitzt. Man versetzt mit Wasser bis zur beginnenden Trübung und kühlt ab. Die ausgefallenen Kristalle werden abgesaugt, mit Wasser nahezu farblos gewaschen und aus Aceton/Wasser umkristallisiert. Die physikalischen Daten und Analysenwerte sind der nachfolgenden Tabelle zu entnehmen.

Für die übrigen, in der nachfolgenden Tabelle aufgeführten erfindungsgemäßen Verbindungen der allgemeinen Formel I (Verbindungen a-i) erfolgt die Darstellung aus der entsprechenden 2-Thioxo-Verbindung in analoger Weise wie oben für die Verbindung (a) beschrieben. Als Lösungsmittel für die Umkristallisation kann dabei, je nach Substanz (siehe Tabelle), auch Toluol, Ethanol, Diethylether/Petrolether oder Diethylether/Ethanol dienen. Die Größe der einzelnen Ansätze lag zwischen 1,5 und 20 mMol.

In den IR-Spektren der Verbindungen der Formel I tritt die Carbamoyl-C=O-Bande des $C_2$ bei 1 660-1 680 $cm^{-1}$ auf; die Lactan-C=O-Bande des $C_4$ ist bei 1 695-1 730 $cm^{-1}$ zu beobachten. Bei den Verbindungen b bis d tritt zusätzlich die Acetyl-C=O-Bande bei 1 740 $cm^{-1}$ auf.

(Siehe Tabelle Seite 9 f.)

Tabelle

Erfindungsgemäße 3-Amino-tetrahydro-1,3-thiazin-2,4-dione der allgemeinen Formel I

| | $R^1$ | $R^2$ | $R^3$ | $R^4$ | $R^5, R^6$ | Schmelzpunkt °C (Umkristallisation aus) | Ausbeute in % der Theorie | Summenformel (Molmasse) | N berechnet gefunden | S |
|---|---|---|---|---|---|---|---|---|---|---|
| a | $CH_3$ | $CH_3$ | $C_6H_5$ | $C_6H_5$ | H | 195-200 (Aceton/Wasser) | 76 | $C_{18}H_{18}N_2O_2S$ (326.42) | 8.58<br>8.57 | 9.82<br>9.56 |
| b | $C_6H_5$ | $CH_3-\underset{\underset{O}{\|}}{C}$ | $C_6H_5$ | $C_6H_5$ | H | 178-181 (Toluol) | 60 | $C_{24}H_{20}N_2O_3S$ (420.50) | 6.66<br>6.59 | 7.62<br>7.82 |
| c | $4-Cl-C_6H_4$ | $CH_3-\underset{\underset{O}{\|}}{C}$ | $C_6H_5$ | $C_6H_5$ | H | 177-179 (Toluol) | 74 | $C_{24}H_{19}ClN_2O_3S$ (450.95) | 6.21<br>6.05 | 7.11<br>7.20 |
| d | $4-NO_2-C_6H_4$ | $CH_3-\underset{\underset{O}{\|}}{C}$ | $C_6H_5$ | $C_6H_5$ | H | 198-199.5 (Toluol) | 71 | $C_{24}H_{19}N_3O_5S$ (461.50) | 9.11<br>8.88 | 6.95<br>6.76 |
| e | $C_6H_5$ | $C_6H_5$ | $C_6H_5$ | $C_6H_5$ | H | 132-136 (Toluol) | 71 | $C_{28}H_{22}N_2O_2S$ (450.56) | 6.22<br>6.02 | 7.12<br>7.32 |
| f | $CH_3$ | $CH_3$ | $4-CH_3-C_6H_4$ | $4-CH_3-C_6H_4$ | H | 190-192 (Toluol) | 65 | $C_{20}H_{22}N_2O_2S$ (354.47) | 7.90<br>7.60 | 9.05<br>8.82 |
| | $R^1, R^2$ | | | | | | | | | |
| g | $-(CH_2)_6-$ | | H | H | H | 68-70 Diethylether/Petrolether | 68 | $C_{10}H_{16}N_2O_2S$ (228.31) | 12.27<br>12.05 | 14.04<br>13.87 |
| h | $-(CH_2)_2-O-(CH_2)_2-$ | | H | H | H | 112-114 Ethanol/Diethylether | 73 | $C_8H_{12}N_2O_3S$ (216.26) | 12.95<br>12.79 | 14.83<br>14.91 |
| i | $-(CH_2)_6-$ | | $4-CH_3-C_6H_4$ | $4-CH_3-C_6H_4$ | H | 73-75 Ethanol | 62 | $C_{24}H_{28}N_2O_2S$ (408.56) | 6.86<br>6.72 | 7.85<br>7.83 |

## Patentansprüche

1. Als Wirkstoff zur Verdickung der Oberhaut geeignete 3-Amino-tetrahydro-1,3-thiazin-2,4-dione der allgemeinen Formel

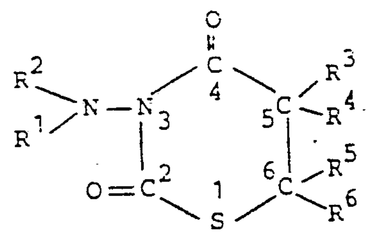

(I)

in der

$R^1$ und $R^2$ unabhängig voneinander H, Alkyl, Hydroxyalkyl, Carboxyalkyl, Halogenalkyl, Cyanoalkyl, Alkoxyalkyl, Cycloalkyl, Alkenyl, Alkinyl, Arylalkyl, Arylalkyl mit substituiertem Arylteil, Aryl, Alkyl-, Halogen-, Nitro-, Alkoxy-, Aryloxy-, Cyanosubstituiertes Aryl, Acyl, Thiazolyl, Thienyl, Benzthiazolyl, 1,3,4-Thiadiazolyl, Oxazolyl, Benzoxazolyl, 1,3,5-Oxadiazolyl, Pyrazolyl, 1,2,4-Triazolyl, Benzimidazolyl, Pyridinyl, Pyrimidinyl, Purinyl, Pyridazinyl, Triazinyl, Benzotriazinyl, Chinolyl, Isochinolyl, Cinnolinyl, Phthalazolyl, Pteridinyl, Chinoxazinyl, Acridinyl bedeuten, oder aber $R^1$ und $R^2$ gemeinsam für Alkyliden, Aryliden und Heterocyclyl-methyliden stehen, wobei der Heterocyclus Furan, Thiophen, Pyrrol, Pyridin, Isoxazol, Thiazol, Imidazol, 1,2,3-Triazol, Pyrazol, Indol, Benzthiazol, Chinolin, Isochinolin, Chinoxalin, Carbazol, Pteridin bedeutet, oder aber $R^1$ und $R^2$ zusammen das Segment $-(CH_2)_n-X-(CH_2)_m-$, mit $X = CH_2$, O, S, NR' (R' = Alkyl, Arylalkyl, Aryl), n = 0 bis 3 und m = 1 bis 3, darstellen, unter der Voraussetzung, daß n nur dann O ist, wenn $X = CH_2$ ist,

$R^3$ einen der Reste H, Alkyl, Cycloalkyl, Carboxyl, Arylalkyl, Aryl oder sustituiertes Aryl darstellt,

$R^4$ die Bedeutung H, Alkyl, Cycloalkyl, Arylalkyl, Aryl oder substituiertes Aryl hat,

$R^5$ einer der Reste H, Alkyl, substituiertes Alkyl, Cycloalkyl, Arylalkyl, Aryl, substituiertes Aryl, 2-Furyl oder substituiertes 2-Furyl ist, und

$R^6$ einen der Reste H, Alkyl, substituiertes Alkyl, Cycloalkyl, Arylalkyl, Aryl oder substituiertes Aryl darstellt.

2. 3-Dimethylamino-5,5-diphenyl-tetrahydro-1,3-thiazin-2,4-dion

3. 3-(N-Acetyl-N-phenyl)-amino-5,5-diphenyl-tetrahydro-1,3-thiazin-2,4-dion

4. 3-[N-Acetyl-N-(4-chlorphenyl)]-amino-5,5-diphenyl-tetrahydro-1,3-thiazin-2,4-dion

5. 3-[N-Acetyl-N-(4-nitrophenyl)]-amino-5,5-diphenyl-tetrahydro-1,3-thiazin-2,4-dion

6. 3-Diphenylamino-5,5-diphenyl-tetrahydro-1,3-thiazin-2,4-dion

7. 3-Dimethylamino-5,5-di-(4-tolyl)-tetrahydro-1,3-thiazin-2,4-dion

8. 3-(1-Perhydroazepinyl)-tetrahydro-1,3-thiazin-2,4-dion

9. 3-(4-Morpholinyl)-tetrahydro-1,3-thiazin-2,4-dion

10. 3-(1-Perhydroazepinyl)-5,5-di-(4-tolyl)-tetrahydro-1,3-thiazin-2,4-dion

11. Hautbehandlungsmittel, enthaltend physiologisch verträgliche Träger- und Zusatzstoffe, gekennzeichnet durch einen Gehalt an mindestens einem als Wirkstoff für die Verdickung der Oberhaut geeigneten 3-Amino-tetrahydro-1,3-thiazin-2,4-dion der allgemeinen Formel I.

12. Mittel nach Anspruch 11, dadurch gekennzeichnet, daß die Verbindungen der Formel I in einer Menge von insgesamt 0,1 bis 5 Gew. %, vorzugsweise 0,5 bis 3 Gew. %, enthalten sind.

13. Hautbehandlungsmittel nach Anspruch 11 oder 12, dadurch gekennzeichnet, daß es ein Hautschutzmittel ist.

14. Hautbehandlungsmittel nach Anspruch 11 oder 12, dadurch gekennzeichnet, daß es ein prophylaktisches Sonnenschutzmittel (Prä-Sun Präparat) ist.

15. Hautbehandlungsmittel nach Anspruch 11 oder 12, dadurch gekennzeichnet, daß es ein prophylaktisches Kälteschutzmittel ist.

16. Hautbehandlungsmittel nach Anspruch 11 oder 12, dadurch gekennzeichnet, daß es ein prophylaktisches Mittel gegen die Hautalterung ist.

17. Mittel nach den Ansprüchen 11 bis 16, dadurch gekennzeichnet, daß es in Form einer Creme vorliegt.

18. Verwendung von Verbindungen der allgemeinen Formel I zur Herstellung von Hautbehandlungsmitteln.

## Claims

1. 3-amino-tetrahydro-1,3-thiazine-2,4-dione suitable as an agent for thickening the epidermis, of the general formula

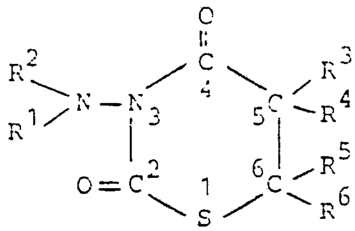

(I)

in which

$R^1$ and $R^2$ independently of each other mean H, alkyl, hydroxyalkyl, carboxyalkyl, halogenalkyl, cyanoalkyl, alkoxyalkyl, cycloalkyl, alkenyl, alkinyl, arylalkyl, arylalkyl with substituted aryl part, aryl, alkyl-, halogen-, nitro-, alkoxy-, aryloxy-, cyano substituted aryl, acyl, thiazolyl, thienyl, benzthiazolyl, 1,3,4-thiadiazolyl, oxazolyl, benzoxazolyl, 1,3,5-oxadiazolyl, pyrazolyl, 1,2,4-triazolyl, benzimidazolyl, pyridinyl, pyrimidinyl, purinyl, pyridazinyl, triazinyl, benzotriazinyl, quinolyl, isoquinolyl, cinnolinyl, phthalazolyl, pteridinyl, quinoxazinyl, acridinyl, or $R^1$ and $R^2$ together mean alkylidene, arylidene and heterocyclylmethylidene, wherein the heterocycle means furan, thiophene, pyrrol, pyridine, isoxazole, thiazole, imidazole, 1,2,3-triazole, pyrazole, indole, benzthiazole, quinoline, isoquinoline, quinoxaline, carbazole, pteridine or $R^1$ and $R^2$ together represent the segment $-(CH_2)_n-X-(CH_2)_m-$, where $X = CH_2$, O, S, NR' (R' = alkyl, arylalkyl, aryl), n = 0 to 3 and m = 1 to 3, provided that n is only 0 if $X = CH_2$,

$R^3$ represents one of the residues H, alkyl, cycloalkyl, carboxyl, arylalkyl, aryl or substituted aryl,

$R^4$ means H, alkyl, cycloalkyl, arylalkyl, aryl or substituted aryl,

$R^5$ is one of the residues H, alkyl, substituted alkyl, cycloalkyl, arylalkyl, aryl, substituted aryl, 2-furyl or substituted 2-furyl, and

$R^6$ represents the residue H, alkyl, substituted alkyl, cycloalkyl, arylalkyl, aryl or substituted aryl.

2. 3-dimethylamino-5,5-diphenyl-tetrahydro-1,3-thiazine-2,4-dione

3. 3-(N-acetyl-N-phenyl)-amino-5,5-diphenyl-tetrahydro-1,3-thiazine-2,4-dione

4. 3-N-acetyl-N-(4-chlorophenyl)-amino-5,5-diphenyl-tetrahydro-1,3-thiazine-2,4-dione

5. 3-N-acetyl-N-(4-nitrophenyl)-amino-5,5-diphenyl-tetrahydro-1,3-thiazine-2,4-dione

6. 3-diphenylamino-5,5-diphenyl-tetrahydro-1,3-thiazine-2,4-dione

7. 3-dimethylamino-5,5-di-(4-tolyl)-tetrahydro-1,3-thiazine-2,4-dione

8. 3-(1-perhydroazepinyl)-tetrahydro-1,3-thiazine-2,4-dione

9. 3-(4-morpholinyl)-tetrahydro-1,3-thiazine-2,4-dione

10. 3-(1-perhydroazepinyl)-5,5-di-(4-tolyl)tetrahydro-1,3-thiazine-2,4-dione

11. Skin treatment composition containing physiologically tolerable carrier and additives characterised in that it contains at least one 3-amino-tetrahydro-1,3-thiazine-2,4-dione suitable as an agent for thickening the epidermis of the general formula I.

12. Medium according to Claim 11, characterised in that the compounds of the formula I are present in a quantity of 0.1 to 5 weight %, preferably 0.5 to 3 weight % in total.

13. Skin treatment composition according to Claim 11 or 12, characterised in that it is a skin protection composition.

14. Skin treatment composition according to Claim 11 or 12, characterized in that it is a prophylactic sun protection composition (pre-sun preparation).

15. Skin treatment composition according to Claim 11 or 12, characterised in that it is a prophylactic cold protection composition.

16. Skin treatment composition according to Claim 11 or 12, characterised in that it is a prophylactic composition against skin ageing.

17. Composition according to any one of Claims 11 to 16, characterised in that it is in the form of a cream.

18. Use of compounds of the general formula I for preparation of skin treatment compositions.

**Revendications**

1. 3-amino-tétrahydro-1,3-thiazine-2,4-dione de formule générale

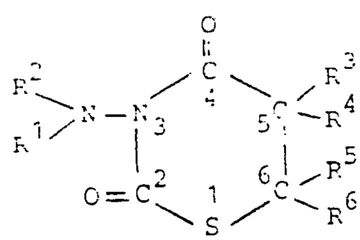

(I)

appropriée comme agent pour épaissir l'épiderme dans laquelle

$R^1$ et $R^2$ désignent indépendamment l'un de l'autre H, alcoyle, hydroxyalcoyle, carboxyalcoyle, halogénalcoyle, cyanoalcoyle, alkoxyalcoyle, cycloalcoyle, alcényle, alcinyle, arylalcoyle, arylalcoyle avec une partie substituée d'aryle, aryle, un aryle substitué par un alcoyle, un halogène, un nitro, un alkoxy, un aryloxy, acyle, thiazoyle, thiényle, benzthiazolyle, 1,3,4-thiadiazolyle, oxazolyle, benzoxazolyle, 1,3,5-oxadiazolyle, pyrazolyle, 1,2,4-triazolyle, benzimidazolyle, pyridinyl, pyrimidinyle, purinyle, pyridazinyle, triazinyle, benzotriazinyle, chinolyle, isochinolyle, cinnolinyle, phthalazolyle, ptéridinyle, chinoxazinyle, acridinyle, ou bien ou $R^1$ et $R^2$ représentent en commun un alcoylidène, un arylidène et un hétérocyclilméthylidène, l'hétérocycle désignant du furanne, du thiopène, du pyrrole, de la pyridine, de l'isoxazol, du thiazol, de l'imidazol, du 1,2,3-triazol, du pyrazol, de l'indol, du benzthiazol, de la chinoline, de l'isochinoline, de la chinoxaline, du carbazol, de la ptéridine ou bien encore $R^1$ et $R^2$ représentent ensemble le segment $-(CH_2)_n-X-(CH_2)_m-$, avec $X = CH_2$, O, S, NR' (R' = alcoyle, arylalcoyle, aryle), $n = 0$ à 3 et $m = 1$ à 3, à la condition que n ne soit 0 que quand $X = CH_2$,

$R^3$ représente l'un des restes H, alcoyle, cycloalcoyle, carboxyle, arylalcoyle, aryle ou aryle substitué.

$R^4$ représente H, alcoyle, cycloalcoyle, arylalcoyle, aryle ou aryle substitué.

$R^5$ représente l'un des restes H, alcoyle, alcoyle substitué, cycloalcoyle, arylalcoyle, aryle, aryle substitué, 2-furyle ou 2-furyle substitué, et

$R^6$ représente l'un des restes H, alcoyle substitué, cycloalcoyle, arylalcoyle, aryle ou aryle substitué.

2. 3-diméthylamino-5,5-diphényl-tétrahydro-1,3-thiazine-2,4-dione.

3. 3-(N-acétyl-N-phényl)-amino-5,5-diphényl-tétrahydro-1,3-thiazine-2,4-dione.

4. 3-[N-acétyl-N-(4-chlorphényl)]-amino-5,5-diphényl-tétrahydro-1,3-thiazine-2,4-dione.

5. 3-[N-acétyl-N-(4-nitrophényl)]-amino-5,5-diphényl-tétrahydro-1,3-thiazine-2,4-dione.

6. 3-diphénylamino-5,5-diphényl-tétrahydro-1,3-thiazine-2,4-dione.

7. 3-diméthylamino-5,5-di(4-tolyl)-tétrahydro-1,3-thiazine-2,4-dione.

8. 3-(1-perhydroazépinyl)-tétrahydro-1,3-thiazine-2,4-dione.

9. 3-(4-morpholinyl)-tétrahydro-1,3-thiazine-2,4-dione.

10. 3-(1-perhydroazépinyl)-5,5-di-(4-tolyl)-tetrahydro-1,3-thiazine-2,4-dione.

11. Composition pour le traitement de la peau contenant de porteurs et des additifs supportables physiologiquement, caractérisé par une teneur d'au moins une 3-amino-tétrahydro-1,3-thiazine-2,4-dione de formule générale I approprié comme agent d'épaississement de l'épiderme.

12. Composition selon la revendication 11, caractérisée en ce que les composés de la formule I sont contenus selon une quantité représentant en tout de 0,1 à 5 % en poids.

13. Composition pour le traitement de la peau selon les revendications 11 et 12, caractérisé en ce qu'il est un agent de protection de la peau.

14. Composition pour le traitement de la peau selon les revendications 11 et 12, caractérisé en ce qu'il est un agent de protection prophylactique contre le soleil (préparation pré-scolaire).

15. Composition pour le traitement de la peau selon les revendications 11 et 12, caractérisé en ce qu'il est un agent de protection prophylactique contre le froid.

16. Composition pour le traitement de la peau selon les revendications 11 et 12, caractérisé en ce qu'il est un agent de proctection prophylactique contre le vieillissement de la peau.

17. Composition selon les revendications 11 à 16, caractérisée en ce qu'il se présente sous la forme d'une crème.

18. Utilisation de composés de la formule générale I pour la fabrication des compositions de traitement de la peau.